# EUROPEAN PATENT APPLICATION

(11) **EP 1 886 675 A1**
(43) Date of publication of application: **13.02.2008**
(21) Application number: 06747070.8
(22) Date of filing: 01.06.2006
(51) Int. Cl.: A61K 9/70, A61K 47/32

(54) **SKIN PATCH**

(30) Priority: 01.06.2005 JP 2005161508
(71) Applicant: Saitama Daiichi Pharmaceutical Co., Ltd., Saitama 344-0057 (JP)
(72) Inventor: KAWAMURA, Naohisa, Kasukabe-shi, Saitama 3440057 (JP); SAWADA, Hidenori, Kasukabe-shi, Saitama 3440057 (JP); SAITOH, Takashi, Kasukabe-shi, Saitama 3440057 (JP); TAKIZAWA, Chihiro, Kasukabe-shi, Saitama 3440057 (JP); TSUCHIYA, Junko, Kasukabe-shi, Saitama 3440057 (JP)
(74) Representative: Maggs, Michael Norman
(86) International application number: PCT/JP2006/310968
(87) International publication number: WO 2006/129745

(57) **Abstract**

A skin patch that can enhance the water resistance and handling easiness and can alleviate any irritation on the user. There is provided a skin patch comprising a flexible backing and, superimposed thereon, a plaster layer wherein the plaster layer contains a styrene-isoprene-styrene block copolymer and has a glass transition temperature (Tg) of -45° to -35°C and a tan δ (lag angle) value, determined at dynamic viscoelasticity measurement (frequency 6.2832 rad/s) at 45°C, of 0.25 or less.

## Description

### TECHNICAL FIELD

The present invention relates to a skin patch, and more particularly, relates to a skin patch having a base layer including a styrene-isoprene-styrene block copolymer.

### BACKGROUND ART

Conventionally, skin patches (for example, plaster) for applying on skin have been extensively used. The adhesion of the skin patches to the skin is important because it allows drugs to transdermally absorb through application to the skin.

Thus, the adhesion of skin patches has been extensively studied. Attempts to use a styrene-isoprene-styrene block copolymer (hereinafter, may be also referred to as SIS block copolymer) as an agglutinant has been made in light of the possibility of production by heat fusion without using a solvent.

For example, a skin patch including indomethacin, a SIS block copolymer, liquid paraffin and polyethylene glycol in the base layer (see, Patent Document 1), a skin patch including a SIS block copolymer, crotamiton, and an antiinflammatory analgesic drug (see, Patent Document 2), and the like have been disclosed.

These skin patches can be produced by heat fusion without using a solvent because a SIS block copolymer was included in the base layer, thus enabling easy and inexpensive production, and a reduced environmental burden can be realized.

Patent Document 1: Japanese Unexamined Patent Application, Publication No. 2001-302502

Patent Document 2: Japanese Unexamined Patent Application, Publication No. H4-321624

### DISCLOSURE OF THE INVENTION

### Problems to be Solved by the Invention

Conventional skin patches as described above are accompanied by the following problems. Firstly, since the aforementioned skin patches have excessively strong self-adhesion (herein, "self-adhesion" is defined as cohesive force, attachment properties and the like between base layer faces of a skin patch, without excluding adhesion to objects other than the base face), the base face is likely to be attached when the release film is peeled, when the skin patch is applied to the skin, or when the patch is applied once again, and thus peeling of the attached faces may be difficult.

Secondly, because the aforementioned skin patches have an excessively strong adhesion to skin, excessive pain may be experienced by a user when peeling a skin patch from the skin.

Thirdly, when the skin gets wet, the adhesion between the skin patch and the skin is reduced, thus the adhesion of the skin patch to the skin is greatly reduced. Therefore, application to the skin may be difficult, or detachment or partial stripping of the skin patch from skin frequently occurs when the skin gets wet due to, for example, sweat, water vapor or the like before or during use.

In connection with self-adhesion, adhesion to the skin, and water resistance (herein, "water resistance" refers to a property that can suppress deterioration in adhesion resulting from wetting of the skin), to date, the accompanying problems could not be entirely solved due to their interrelation.

Accordingly, an object of the present invention is to provide a skin patch in which a SIS block copolymer is used as an agglutinant, and which can improve the water resistance and handleability, and can reduce the amount of stimulation a user experiences.

### Means for Solving the Problems

The present inventors thoroughly investigated the problems as described above, and found that water resistance and handleability can be improved, and stimulation that a user experiences can be reduced by allowing tanδ (angle of lag) and a glass transition temperature (Tg) of the base as determined by measurement of the dynamic viscoelasticity to fall within a predetermined range. Accordingly, the present invention was accomplished.

More specifically, the present invention provides the following aspects.

In a first aspect of the invention, a skin patch including a flexible backing, and a base layer laminated on the backing is provided, wherein the base layer includes a styrene-isoprene-styrene block copolymer, having a glass transition temperature (Tg) of no less than -45°C and no greater than -35°C, and having a tanδ (angle of lag) value determined by measurement of dynamic viscoelasticity (frequency: 6.2832 (rad/s)) at 45°C being no greater than 0.25.

In a second aspect of the skin patch according to the first aspect of the present invention, the tanδ (angle of lag) value is no less than 0.12 and no greater than 0.25.

In a third aspect of the skin patch according to the second aspect of the present invention, the tanδ (angle of lag) value is no less than 0.15 and no greater than 0.25.

In a fourth aspect of the skin patch according to any one of the first to the third aspects of the present invention, the base layer further includes a tackifier, and a plasticizer.

In a fifth aspect of skin patch according to the fourth aspect of the present invention, the content of the styrene-isoprene-styrene block copolymer is no less than 10% by weight and no greater than 40% by weight; the content of the tackifier is from 10 to 35% by weight; and the content of the plasticizer is no less than 20% by weight and no greater than 60% by weight based on the weight of the entire base layer.

In a sixth aspect of the skin patch according to any one of the first to the fifth aspects of the present invention, the styrene-isoprene-styrene block copolymer has a weight ratio of styrene to isoprene (styrene/isoprene) no less than 20/80 and no greater than 25/75.

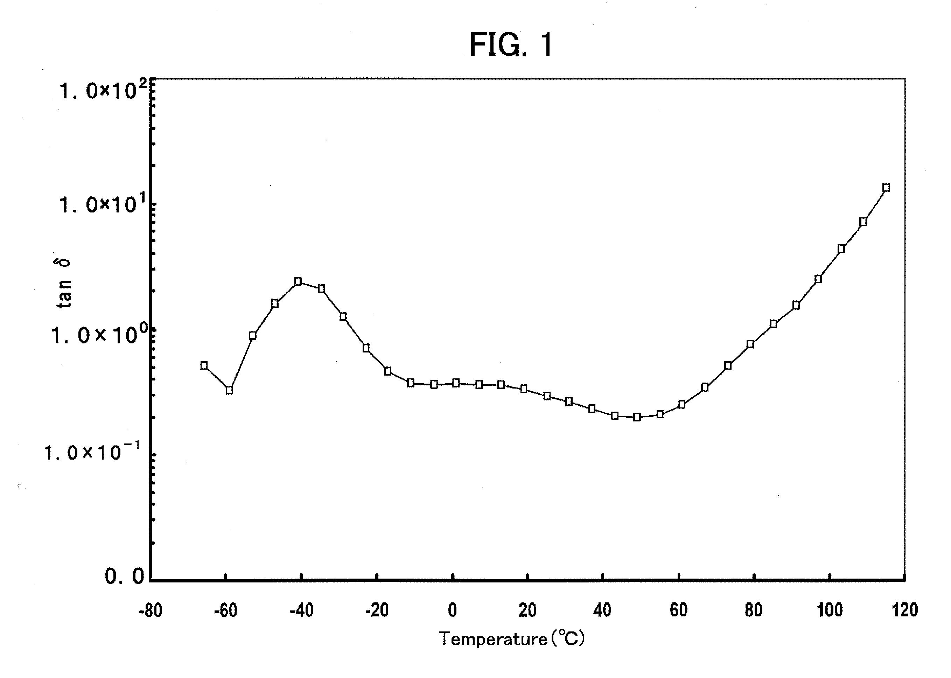

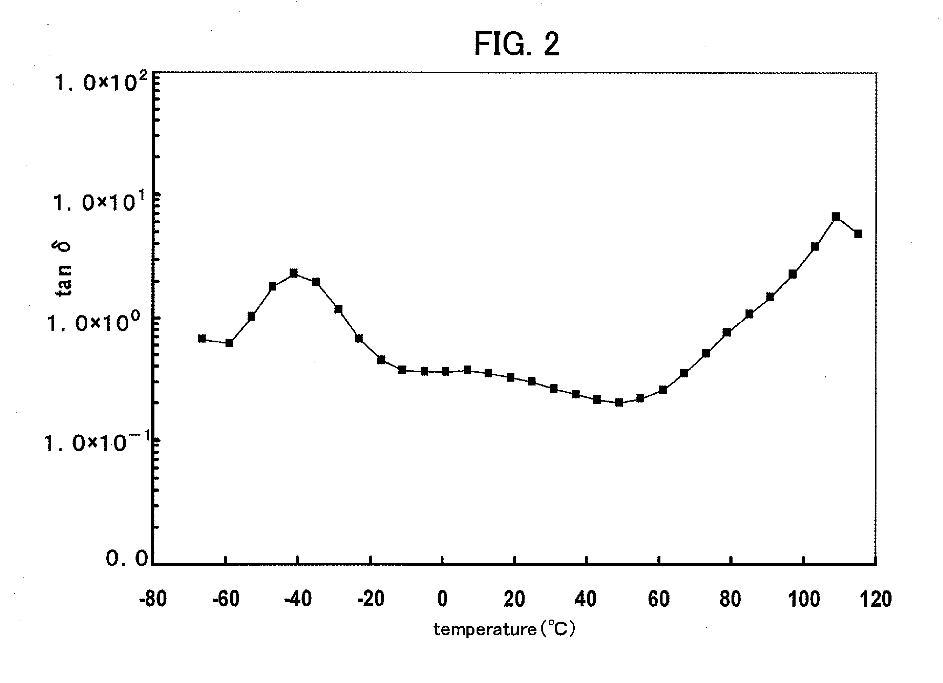

In a seventh aspect of the skin patch according to the present invention, a skin patch including a flexible backing, and a base layer laminated on this backing is provided, wherein the base layer includes a styrene-isoprene-styrene block copolymer, and exhibits temperature dependency of tanδ (angle of lag) that is substantially similar to the illustration of temperature dependency of tanδ (angle of lag) shown in Fig. 1 or Fig. 2 attained by measurement of dynamic viscoelasticity (frequency: 6.2832 (rad/s)).

According to the seventh aspect of the invention, the base exhibits temperature dependency of tanδ (angle of lag) that is substantially similar to the illustration of temperature dependency of tanδ (angle of lag) shown in Fig. 1 or Fig. 2. The illustration (Figs. 1 and 2) of temperature dependency of tanδ (angle of lag) specifically represents the values shown in Table 1.

**Table 1**

| Figure 1 | | Figure 2 | |
|---|---|---|---|
| Temperature (°C) | tanδ | Temperature (°C) | tanδ |
| -65.57 | 5.18 x 10⁻¹ | -66.26 | 6.72 x 10⁻¹ |
| -58.8 | 3.32 x 10⁻¹ | -58.74 | 6.17 x 10⁻¹ |
| -52.83 | 8.80 x 10⁻¹ | -52.79 | 1.03 x 10⁰ |
| -46.87 | 1.56 x 10⁰ | -46.86 | 1.81 x 10⁰ |
| -40.88 | 2.37 x 10⁰ | -40.87 | 2.27 x 10⁰ |
| -34.87 | 2.08 x 10⁰ | -34.86 | 1.94 x 10⁰ |
| -28.88 | 1.24 x 10⁰ | -28.87 | 1.17 x 10⁰ |
| -22.88 | 7.00 x 10⁻¹ | -22.87 | 6.79 x 10⁻¹ |
| -16.88 | 4.60 x 10⁻¹ | -16.87 | 4.51 x 10⁻¹ |
| -10.89 | 3.77 x 10⁻¹ | -10.88 | 3.74 x 10⁻¹ |
| -4.88 | 3.67 x 10⁻¹ | -4.88 | 3.64 x 10⁻¹ |
| 1.08 | 3.69 x 10⁻¹ | 1.12 | 3.68 x 10⁻¹ |
| 7.12 | 3.64 x 10⁻¹ | 7.11 | 3.69 x 10⁻¹ |
| 13.1 | 3.60 x 10⁻¹ | 13.12 | 3.53 x 10⁻¹ |
| 19.11 | 3.37 x 10⁻¹ | 19.11 | 3.31 x 10⁻¹ |
| 25.12 | 2.94 x 10⁻¹ | 25.07 | 3.05 x 10⁻¹ |
| 31.1 | 2.68 x 10⁻¹ | 31.11 | 2.63 x 10⁻¹ |
| 37.12 | 2.34 x 10⁻¹ | 37.1 | 2.38 x 10⁻¹ |
| 43.11 | 2.07 x 10⁻¹ | 43.12 | 2.11 x 10⁻¹ |
| 49.12 | 2.00 x 10⁻¹ | 49.09 | 2.02 x 10⁻¹ |
| 55.1 | 2.08 x 10⁻¹ | 55.12 | 2.17 x 10⁻¹ |
| 61.12 | 2.50 x 10⁻¹ | 61.1 | 2.60 x 10⁻¹ |
| 67.11 | 3.42 x 10⁻¹ | 67.11 | 3.55 x 10⁻¹ |
| 73.11 | 5.13 x 10⁻¹ | 73.09 | 5.18 x 10⁻¹ |
| 79.11 | 7.67 x 10⁻¹ | 79.11 | 7.69 x 10⁻¹ |
| 85.07 | 1.11 x 10⁰ | 85.1 | 1.09 x 10⁰ |
| 91.11 | 1.54 x 10⁰ | 91.11 | 1.50 x 10⁰ |
| 97.09 | 2.50 x 10⁰ | 97.1 | 2.31 x 10⁰ |
| 103.09 | 4.30 x 10⁰ | 103.1 | 3.75 x 10⁰ |
| 109.1 | 6.98 x 10⁰ | 109.1 | 6.54 x 10⁰ |
| 115.07 | 1.34 x 10¹ | 115.05 | 4.79 x 10⁰ |

Furthermore, the skin patch of the present invention, which exhibits temperature dependency of tanδ (angle of lag) that is substantially similar to the illustration of temperature dependency of tanδ (angle of lag) shown in Fig. 1 or Fig. 2, can be also characterized as a skin patch that exhibits temperature dependency of dynamic shear modulus (G') that is substantially similar to the illustration of temperature dependency of dynamic shear modulus G' shown in Fig. 3 determined by measurement of the dynamic viscoelasticity. The illustration (Fig. 3) of the temperature dependency of the dynamic shear modulus (G') represents the values shown in Table 2.

**Table 2**

| Figure 1 | | Figure 2 | |
|---|---|---|---|
| Temperature (°C) | G' (Pa) | Temperature (°C) | G' (Pa) |
| -65.57 | 3.85 x 10⁸ | -66.26 | 1.27 x 10⁷ |
| -58.8 | 3.29 x 10⁸ | -58.74 | 1.30 x 10⁷ |
| -52.83 | 8.78 x 10⁷ | -52.79 | 6.84 x 10⁶ |
| -46.87 | 1.32 x 10⁷ | -46.86 | 2.95 x 10⁶ |
| -40.88 | 1.48 x 10⁶ | -40.87 | 1.53 x 10⁶ |
| -34.87 | 2.85 x 10⁵ | -34.86 | 3.19 x 10⁵ |
| -28.88 | 1.08 x 10⁵ | -28.87 | 1.23 x 10⁵ |
| -22.88 | 5.45 x 10⁴ | -22.87 | 6.28 x 10⁴ |
| -16.88 | 3.55 x 10⁴ | -16.87 | 3.96 x 10⁴ |
| -10.89 | 2.80 x 10⁴ | -10.88 | 3.08 x 10⁴ |
| -4.88 | 2.42 x 10⁴ | -4.88 | 2.66 x 10⁴ |
| 1.08 | 2.09 x 10⁴ | 1.12 | 2.30 x 10⁴ |
| 7.12 | 1.84 x 10⁴ | 7.11 | 2.01 x 10⁴ |
| 13.1 | 1.64 x 10⁴ | 13.12 | 1.78 x 10⁴ |
| 19.11 | 1.48 x 10⁴ | 19.11 | 1.61 x 10⁴ |
| 25.12 | 1.38 x 10⁴ | 25.07 | 1.49 x 10⁴ |
| 31.1 | 1.31 x 10⁴ | 31.11 | 1.40 x 10⁴ |
| 37.12 | 1.26 x 10⁴ | 37.1 | 1.35 x 10⁴ |
| 43.11 | 1.23 x 10⁴ | 43.12 | 1.31 x 10⁴ |
| 49.12 | 1.20 x 10⁴ | 49.09 | 1.28 x 10⁴ |
| 55.1 | 1.16 x 10⁴ | 55.12 | 1.24 x 10⁴ |
| 61.12 | 1.09 x 10⁴ | 61.1 | 1.16 x 10⁴ |
| 67.11 | 9.72 x 10³ | 67.11 | 1.04 x 10⁴ |
| 73.11 | 7.85 x 10³ | 73.09 | 8.39 x 10³ |
| 79.11 | 5.44 x 10³ | 79.11 | 5.92 x 10³ |
| 85.07 | 3.19 x 10³ | 85.1 | 3.50 x 10³ |
| 91.11 | 1.60 x 10³ | 91.11 | 1.84 x 10³ |
| 97.09 | 6.39 x 10² | 97.1 | 7.95 x 10² |
| 103.09 | 2.08 x 10² | 103.1 | 2.66 x 10² |
| 109.1 | 6.42 x 10¹ | 109.1 | 7.36 x 10¹ |
| 115.07 | 1.70 x 10¹ | 115.05 | 4.56 x 10¹ |

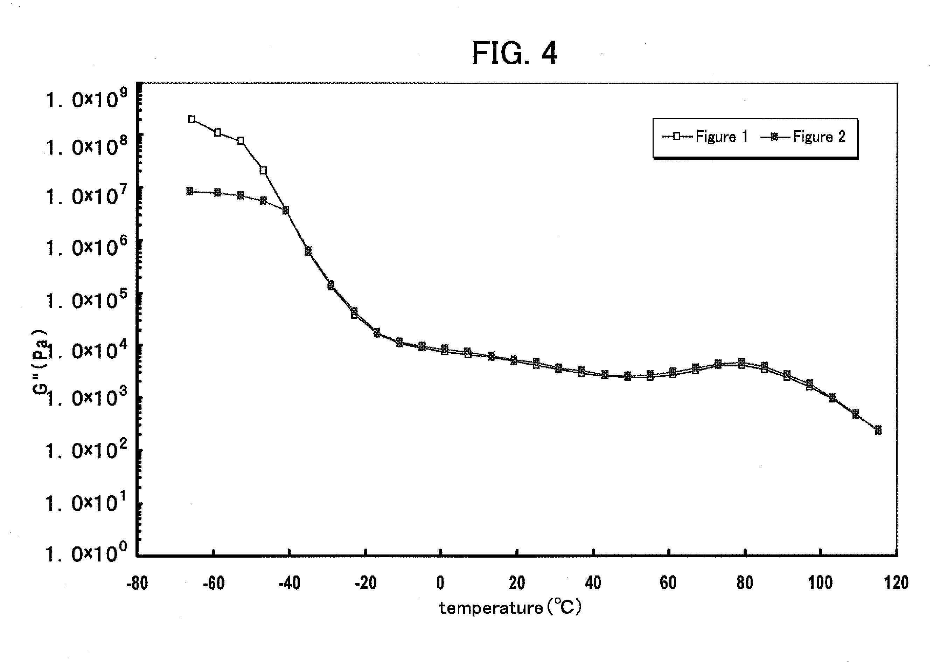

In addition, the skin patch of present invention, which exhibits temperature dependency of tanδ (angle of lag) that is substantially similar to the illustration of temperature dependency of tanδ (angle of lag) shown in Fig. 1 or Fig. 2, can be also characterized by being a skin patch that exhibits temperature dependency of dynamic loss modulus (G") that is substantially similar to the illustration of temperature dependency of dynamic loss modulus G" shown in Fig. 4 determined by measurement of dynamic viscoelasticity. Moreover, the illustration (Fig. 4) of the dynamic loss modulus (G") represents the values shown in Table 3.

**Table 3**

| Figure 1 | | Figure 2 | |
|---|---|---|---|
| Temperature (°C) | G" (Pa) | Temperature (°C) | G" (Pa) |
| -65.57 | 2.00 x 10⁸ | -66.26 | 8.54 x 10⁶ |
| -58.8 | 1.09 x 10⁸ | -58.74 | 8.01 x 10⁶ |
| -52.83 | 7.73 x 10⁷ | -52.79 | 7.04 x 10⁶ |
| -46.87 | 2.06 x 10⁷ | -46.86 | 5.33 x 10⁶ |
| -40.88 | 3.50 x 10⁶ | -40.87 | 3.49 x 10⁶ |
| -34.87 | 5.93 x 10⁵ | -34.86 | 6.20 x 10⁵ |
| -28.88 | 1.34 x 10⁵ | -28.87 | 1.44 x 10⁵ |
| -22.88 | 3.81 x 10⁴ | -22.87 | 4.27 x 10⁴ |
| -16.88 | 1.63 x 10⁴ | -16.87 | 1.79 x 10⁴ |
| -10.89 | 1.05 x 10⁴ | -10.88 | 1.15 x 10⁴ |
| -4.88 | 8.90 x 10³ | -4.88 | 9.68 x 10³ |
| 1.08 | 7.73 x 10³ | 1.12 | 8.46 x 10³ |
| 7.12 | 6.71 x 10³ | 7.11 | 7.41 x 10³ |
| 13.1 | 5.88 x 10³ | 13.12 | 6.27 x 10³ |
| 19.11 | 5.00 x 10³ | 19.11 | 5.32 x 10³ |
| 25.12 | 4.06 x 10³ | 25.07 | 4.54 x 10³ |
| 31.1 | 3.50 x 10³ | 31.11 | 3.69 x 10³ |
| 37.12 | 2.96 x 10³ | 37.1 | 3.21 x 10³ |
| 43.11 | 2.54 x 10³ | 43.12 | 2.77 x 10³ |
| 49.12 | 2.40 x 10³ | 49.09 | 2.59 x 10³ |
| 55.1 | 2.41 x 10³ | 55.12 | 2.68 x 10³ |
| 61.12 | 2.73 x 10³ | 61.1 | 3.03 x 10³ |
| 67.11 | 3.33 x 10³ | 67.11 | 3.69 x 10³ |
| 73.11 | 4.03 x 10³ | 73.09 | 4.35 x 10³ |
| 79.11 | 4.17 x 10³ | 79.11 | 4.55 x 10³ |
| 85.07 | 3.53 x 10³ | 85.1 | 3.81 x 10³ |
| 91.11 | 2.46 x 10³ | 91.11 | 2.76 x 10³ |
| 97.09 | 1.60 x 10³ | 97.1 | 1.84 x 10³ |
| 103.09 | 8.96 x 10² | 103.1 | 9.97 x 10² |
| 109.1 | 4.48 x 10² | 109.1 | 4.81 x 10² |
| 115.07 | 2.28 x 10² | 115.05 | 2.19 x 10² |

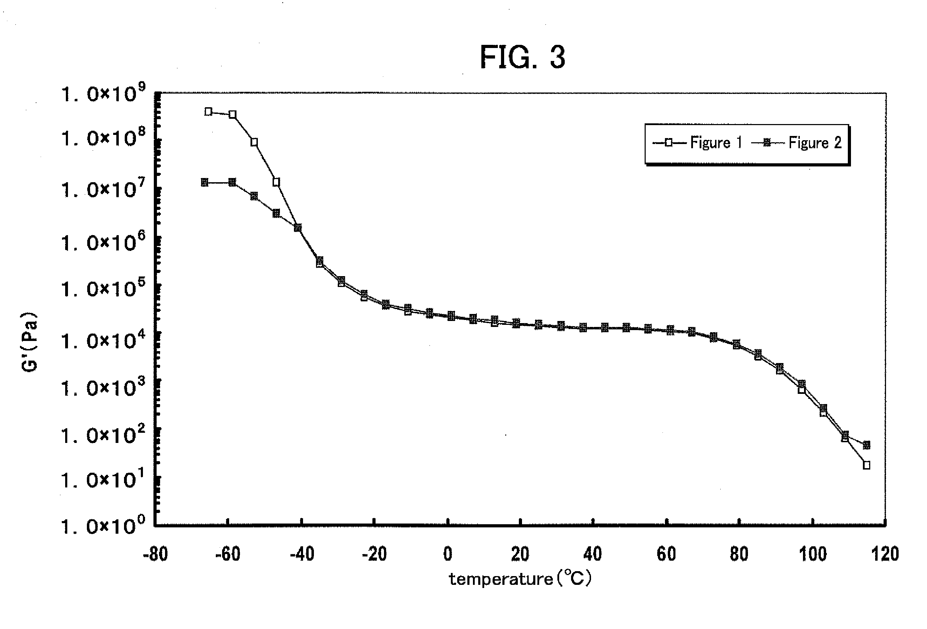

The illustration of temperature dependency of tanδ (angle of lag) shown in Fig. 1 or Fig. 2 may be determined from the illustration (Fig. 3) of temperature dependency of the dynamic shear modulus (G'), and the illustration (Fig. 4) of temperature dependency of the dynamic loss modulus (G'').

### Effects of the Invention

According to the present invention, because the base including the SIS block copolymer has a glass transition temperature (Tg) of no less than -45°C and no greater than - 35°C, and has a tanδ (angle of lag) value determined by measurement of dynamic viscoelasticity (frequency: 6.2832 (rad/s)) at 45°C being no greater than 0.25, water resistance and handleability can be improved, and stimulation that a user experiences can be reduced.

### BRIEF DESCRIPTION OF THE DRAWINGS

Fig. 1 shows an illustration of temperature dependency of tanδ (angle of lag) determined by measurement of dynamic viscoelasticity;
Fig. 2 shows an illustration of temperature dependency of tanδ (angle of lag) determined by measurement of dynamic viscoelasticity;
Fig. 3 shows an illustration of temperature dependency of dynamic shear modulus (G') determined by measurement of dynamic viscoelasticity; and
Fig. 4 shows an illustration of temperature dependency of dynamic loss modulus (G") determined by measurement of dynamic viscoelasticity.

### PREFERRED MODE FOR CARRYING OUT THE INVENTION

Hereinafter, a first example of an embodiment of the present invention will be explained; however, the present invention is not limited to the following embodiment.

The skin patch of the present invention includes a flexible backing, and a base layer laminated on the backing. Herein, the skin patch of the present invention is principally for application to skin, and may include plasters, cataplasms, tapes, adhesive plasters, sheets, wound dressings, cosmetic facial masks and the like, however, industrial tapes are not included.

### Base Layer

The base layer that constitutes the skin patch includes a SIS block copolymer as an essential component, and preferably, further includes a tackifier and a plasticizer.

### SIS Block Copolymer

The SIS block copolymer is a type of rubber-based agglutinant, and belongs to an A-B-A type polymer, which is a styrene thermoplastic elastomer having a molecular structure in which "A" as end blocks represents polystyrene, and "B" as intermediate block represents polyisoprene.

The SIS block copolymer which may be used in the present invention is not particularly limited, but in general, may have a solution viscosity (MPa·s [cps], 25°C) of about 100 to 3000, and have a weight ratio of styrene and isoprene of 10/90 to 30/70. Preferably, the weight ratio of styrene and isoprene (styrene/isoprene) is 20/80 to 25/75. By using such a SIS block copolymer having a high weight ratio of styrene, preparation of the base is facilitated.

Specifically, the following commercial SIS based resins can be used. For example, one having a styrene/rubber ratio (% by weight) of 15/85 and a solution viscosity (MPa·s [cps], 25°C) of 1,500 (trade name: Kraton D-1107), one having a styrene/rubber ratio (% by weight) of 15/85 and a solution viscosity (MPa·s [cps], 25°C) of 900 (trade name: Kraton D-1112), one having a styrene/rubber ratio (% by weight) of 17/83 and a solution viscosity (MPa·s [cps], 25°C) of 500 (trade name: Kraton D-1117P), one having a styrene/rubber ratio (% by weight) of 22/78 (trade name: Kraton D-KX401), one having a styrene/rubber ratio (% by weight) of 16/84 (trade name: Kraton D-KX406), one having a styrene/rubber ratio (% by weight) of 30/70 and a solution viscosity (MPa·s [cps], 25°C) of 300 (trade name: Kraton D-1125x), one having a styrene/rubber ratio (% by weight) of 10/90 and a solution viscosity (MPa·s [cps], 25°C) of 2,500 (trade name: Kraton D-1320x) are included (all manufactured by Kraton Polymer Japan Co., Ltd.). The SIS block copolymer used in the present invention may include one, or two or more of these products, and one having a styrene/rubber ratio (% by weight) of 22/78 (trade name: Kraton D-KX401) is preferred.

The content of the SIS block copolymer is not particularly limited, and it is preferably 10 to 40% by weight based on the weight of the entire base. An exceedingly low content of the SIS block copolymer is not preferred because the cohesion becomes insufficient. In contrast, an exceedingly high content is also not preferred because adhesion to the skin becomes insufficient.

### Tackifier

The tackifier which may be used in the present invention is not particularly limited, and for example, alicyclic saturated hydrocarbon resins (synthetic petroleum resin) as well as rosin ester derivatives, terpene based resins, phenolic resins and the like are preferred.

The alicyclic saturated hydrocarbon resin is not particularly limited, and for example, "ARKON P-100 (trade name)" (manufactured by Arakawa Chemical Industries, Ltd.) and the like may be exemplified. The rosin ester derivative is not particularly limited, and for example, "Ester Gum H (trade name)" (manufactured by Arakawa Chemical Industries, Ltd.), "KE-311 (trade name)" (manufactured by Arakawa Chemical Industries, Ltd.), "KE-100 (trade name)" (manufactured by Arakawa Chemical Industries, Ltd.), and the like are exemplified. The terpene based resin is not particularly limited, and for example, "YS resin (trade name)" (manufactured by YASUHARA CHEMICAL Co., Ltd.) and the like may be exemplified. The tackifier used in the present invention may be, for example, may include any one, two or more of these agents.

The content of the tackifier is not particularly limited, and is preferably 10 to 35% by weight based on the weight of the entire base. When the content of the tackifier is too low, the adhesion becomes insufficient. In contrast, when the content of the tackifier is too high, the adhesion becomes excessively great, thus a user may experience excessive pain when a skin patch is peeled from the skin.

### Plasticizer

The plasticizer which is optionally employed in the present invention is not particularly limited, and for example, liquid paraffin, hydrogenated oil, hydrogenated castor oil, higher alcohol such as octyldodecanol, squalane, squalene, castor oil, liquid rubber (polybutene), fatty acid esters such as isopropyl myristate, and the like may be exemplified. The plasticizer used in the present invention may be, for example, may include any one, two or more of these. Moreover, among these, liquid paraffin, hydrogenated oil, hydrogenated castor oil are preferred.

The content of the plasticizer is preferably 20 to 60% by weight based on the weight of the entire base. When the content of the plasticizer is too low, the adhesion becomes insufficient because the base layer is excessively hardened. In contrast, when the content of the plasticizer is too high, the base layer is excessively softened, whereby stickiness may result, and thus a user may experience excessive pain when peeling a skin patch from the skin, or residual base is likely to remain. The content of the plasticizer is more preferably 25 to 50% by weight, and still more preferably 30 to 50% by weight.

### Optional Ingredient

The base layer that constitutes the skin patch may contain, as needed, any optional ingredients such as medicinal agents, excipients, anti-oxidizing agents, drug solubilizers, transdermal absorption promoting agents, flavors, colorant and the like, in addition to the ingredients described above. The optional ingredient may include any one, two or more of these. Medicinal Agent

As the medicinal agent, for example, general anesthetics, hypnotics, analgesics, antiphlogistic analgetics, steroid hormonal drugs, analeptic stimulant drugs, drugs for psychoneurosis, topical anesthetics, skeletal muscle relaxants, drugs for autonomic nerve, anti-allergic drugs, antihistamic drugs, cardiac stimulants, drugs for arrhythmia, diuretic drugs, hypotensive drugs, vasoconstrictors, vasodilators, calcium antagonists, anti-bacteriocides, drugs for parasitism-developed skin diseases, skin softening agents, antibiotics, antidotes, antitussive drugs, antipruritic drugs, hypnotics, spiritual exaltation agents, antiasthmatics, hormone secretion accelerators, antiulcer drugs, antitumor drugs, vitamins, agents having a whitening effect such as ingredients for beautiful skin, and the like may be included.

First, when the intended use of the skin patch of the present invention is for a topically acting skin patch, the following agents may be included therein, for example, an antiphlogistic analgetic such as indomethacin, ketoprofen, flurbiprofen, loxoprofen, loxoprofen sodium, piroxicam, meloxicam, ketorolac, felbinac, diclofenac, diclofenac sodium, or the like. Among these, at least one selected from the group consisting of indomethacin, ketoprofen, felbinac, loxoprofen, diclofenac, and salts thereof is preferable. The content of the agent is not particularly limited, and may be generally approximately 0.1 to 20% by weight based on the weight of the entire base layer.

### Excipient

The excipient is not particularly limited, and examples thereof include silicon compounds such as silicate anhydride, light silicate anhydride and hydrous silicic acid, cellulose derivatives such as ethyl cellulose, methyl cellulose, hydroxypropyl cellulose and hydroxypropylmethyl cellulose, water soluble polymers such as polyvinylalcohol, aluminum compounds such as dry aluminum hydroxide gel and hydrous aluminum silicate, kaolin, titanium oxide, and the like.

### Anti-oxidizing Agent

The anti-oxidizing agent is not particularly limited, and for example, dibutylhydroxytoluene, ascorbic acid, tocopherol, tocopherol ester derivatives, butylhydroxyanisole, 2-mercaptobenzimidazole, and the like may be exemplified. Solubilizer and Transdermal Absorption Promoting Agent of Drug

The solubilizer, and the transdermal absorption promoting agent of the drug are not particularly limited, and polyhydric alcohols such as polyethylene glycol (average molecular weight: 200 to 30000), glycerin, ethylene glycol, and diethylene glycol, fatty acids such as oleic acid, isostearic acid and citric acid, fatty acid esters such as isopropyl myristate, isopropyl palmitate, and diisopropyl adipate, fatty acid polyhydric alcohol esters such as caprylic acid monoglyceride, caprylic acid triglyceride, and sorbitan fatty acid esters, terpenes such as menthol, menthol derivatives, peppermint oil, and limonene, N-methyl-2-pyrrolidone, crotamiton, polyvinylalcohol, and the like are exemplified.

### Backing

The backing used in the present invention is not particularly limited, and stretch or nonstretch knits, or nonwoven fabrics of polyethylene, polypropylene or the like, films of polyethylene, polypropylene, ethylene acetate vinyl copolymer, vinyl chloride or the like, or foam backings of urethane, polyurethane or the like can be used. The backing may include any one, two or more of these materials.

### Liner

The skin patch of the present invention has a flexible backing and a base layer provided by applying a base on a first face of the backing, and additionally is generally provided in a form in which a releasable liner is laminated on the base layer.

As the releasable liner, which is typically used in the present invention, a film of polyethylene, polypropylene, ethylene vinyl acetate copolymer, vinyl chloride or the like, a metal film prepared by aluminum vapor deposition or the like may be exemplified, and the liner surface may be subjected to a release treatment such as a silicon treatment or the like may be included. As the "releasable liner" used in the present invention, for example, those having a linear or curved cut, those in which two or more liners overlap in part, those having a turned edge are employed in view of easy release thereof.

### Method of Manufacture

### Base Preparation

For the skin patch of the present invention, a base is prepared by mixing a raw material composition which includes a SIS block copolymer, while stirring in a Henschel mixer (registered trade name) or the like under a shear loading condition (stirring speed: 50 rpm to 1500 rpm) from initiation of fusion until a fused state is reached (from room temperature to 250°C), additionally, mixing of the fused state may be applied if needed. This method enables preparation of a base having physical parameters according to the present invention.

Mixing with stirring is not particularly limited, and may be carried out from initiation of the fusion until the fused state is reached. The temperature during mixing with stirring is preferably no less than 50°C. The stirring is preferably conducted concurrently with shear loading. In this case, stirring speed is preferably no less than 50 rpm, and more preferably no less than 100 rpm.

The apparatus used for mixing by stirring may be, for example, a Henschel mixer (registered trade name), a kneader, an open roll, a mixing roll, an internal mixer, a banbury mixer, a plast mill, a biaxial kneader, an extrusion kneader, or the like. Among these, in light of ease in stirring at a high speed, a Henschel mixer (registered trade name) is preferred.

### Production of Skin Patch Plaster

The thickness of the base layer applied on the backing of the skin patch of the present invention is preferably 90 µm to 250 µm. When the thickness of the base layer is too great, it is likely to be stripped during use as a result of contact with clothes or the like. In contrast, when the thickness is too small, backing provided to the skin patch is lost, thus application error is likely to result.

The method of manufacturing the skin patch of the present invention is not limited to the procedures as described above, and the temperature conditions, rate of stirring, stirring time and the like may be regulated depending on the machine used for production, production scale, and the like.

According to the present method of manufacture, a base having a glass transition temperature (Tg) of no less than - 45°C and no greater than -35°C, and a tanδ (angle of lag) value determined by measurement of dynamic viscoelasticity (frequency: 6.2832 (rad/s)) at 45°C being no greater than 0.25 can be readily produced.
Measurement of Tanδ (angle of lag) and Glass Transition

### Temperature

Tanδ (angle of lag) is a physical characteristic value derived from dynamic loss modulus (G") and dynamic shear modulus (storage modulus of elasticity) (G') according to the following formula: tanδ (angle of lag) = G''/G'.

The glass transition temperature is defined as a temperature at which a physical property of a glassy state suddenly changes, and it can be determined from a tanδ curve profile or the like with respect to the change in temperature.

The measurement of tanδ (angle of lag) and the glass transition temperature may be carried out according to the following procedures using, for example, Rheometric Dynamic Analyzer as an apparatus for the measurement.

First, the base is scraped from the skin patch, and then from 50 mg to 75 mg of the base is sandwiched in a cylindrical jig in the aforementioned apparatus. Next, the stress generated when a strain, at a pre-determined frequency, is applied while changing the temperature is detected. Based on the results of the detected value, the dynamic loss modulus (G") and the dynamic shear modulus (storage modulus of elasticity) (G') are calculated to determined the tanδ (angle of lag) value. Furthermore, the glass transition temperature (Tg value) can be specified from the profile curve of tanδ, in terms of the temperature at the vertex (value on the abscissa).

The base that constitutes the skin patch of the present invention has a glass transition temperature (Tg) of no less than -45°C and no greater than -35°C, and a tanδ (angle of lag) value determined by measurement of dynamic viscoelasticity (frequency: 6.2832 (rad/s)) at 45°C being no greater than 0.25. The glass transition temperature (Tg) is preferably no less than -43°C and no greater than -35°C. In addition, the tanδ (angle of lag) value at 45°C is preferably no less than 0.12 and no greater than 0.25, and more preferably no less than 0.15 and no greater than 0.25 because the water resistance and handleability can be further improved, and the stimulation experienced by a user can be further reduced.

The water resistance, the handleability and the skin stimulatory property of the skin patch are inferred to correlate to the coordinate of flexion point on the illustration of temperature dependency of tanδ (angle of lag) of the base. In other words, by altering the temperature at the glass transition point (Tg) corresponding to the coordinate of the flexion point on the illustration of temperature dependency of tanδ (angle of lag), and tanδ (angle of lag) at 45°C, the water resistance, the handleability, and the skin stimulatory property of the skin patch can be regulated.

Also, tanδ of the base at 100°C is preferably no less than 2.5 and no greater than 4.0 because the mixture can be readily sheared by stirring of the mixture (at about 100 to 150°C) in the procedure of preparing the base.

The base that constitutes the skin patch of the present invention has a tanδ (angle of lag) value at 32°C of preferably no less than 0.25 and no greater than 0.35, and more preferably no less than 0.25 and no greater than 0.30.

### EXAMPLES

Next, the present invention will be specifically explained by way of Examples, but the present invention is not in any way limited thereto.

### Example 1

Into a Henschel mixer (registered trade name, manufactured by Mitsui Mining Co., Ltd.,) 3870 g of liquid paraffin (MORESCO WHITE P-350P; manufactured by Matsumura Oil Research Corp.) as a plasticizer, 90 g of dibutylhydroxytoluene as an anti-oxidizing agent, 2250 g of a pre-heated "Ester Gum H (trade name)" (manufactured by Arakawa Chemical Industries, Ltd.) as a tackifier, were added and mixed at 200 rpm and a temperature of 100°C.

To this mixture 2250 g of a SIS block copolymer (D-KX401CS; manufactured by Kraton Polymer Japan Co., Ltd.) having a weight ratio of styrene and isoprene (styrene/isoprene) being 22/78, was added and the mixture was dissolved by raising the temperature to 150°C while shearing at 800 rpm and a temperature of 100°C.

Subsequently, the resulting solution was stirred while shearing at 800 rpm for 10 min. To the stirred mixture a drug solubilizer as well as 270 g of 1-menthol, and 270 g of N-methyl-2-pyrrolidone as a transdermal absorption-promoting agent were added followed by mixing with stirring to prepare a base.

Subsequently, the resulting base was applied to a knit (made with polyester), having a mass per unit area of 100 g/m², to a thickness of 150 µm. A skin patch (plaster) was produced by laminating on the base a liner (made of PET) which had been subjected to silicon treatment.

### Example 2

A skin patch (plaster) was produced by preparing a base by a similar procedure to Example 1 except that the amount of liquid paraffin added was 3780 g, and the amount of 1-menthol added was 90 g, and 180 g of hydrogenated oil and 90 g of diclofenac sodium were added.

### Example 3

A skin patch (plaster) was produced by preparing a base by a similar procedure to Example 2 except that the amount of liquid paraffin added was 3285 g, and the amount of N-methyl-2-pyrrolidone added was 360 g, and 360 g of isostearic acid and 45g of citric acid were added.

### Example 4

A skin patch (plaster) was produced by preparing a base by a similar procedure to Example 1 except that a SIS block copolymer (D-1107; manufactured by Kraton Polymer Japan Co., Ltd.) having a weight ratio (styrene/isoprene) of 15/85 of styrene to isoprene was added in place of "D-KX401CS (trade name)", and "KE-100 (trade name)" (manufactured by Arakawa Chemical Industries, Ltd.) was added in place of the ester gum H.

### Example 5

A skin patch (plaster) was produced by preparing a base by a similar procedure to Example 4 except that the amount of liquid paraffin added was 3780 g, and "ester gum H (trade name)" (manufactured by Arakawa Chemical Industries, Ltd.) was added in place of "KE-100 (trade name)", and that 90 g of diclofenac sodium was added.

### Example 6

A skin patch (plaster) was produced by preparing a base by a similar procedure to Example 1 except that the amount of liquid paraffin added was 3780 g, and 90 g of ketoprofen was added.

### Example 7

A skin patch (plaster) was produced by preparing a base by a similar procedure to Example 6 except that "ARKON P-100 (trade name)" (manufactured by Arakawa Chemical Industries, Ltd.) was added in place of "Ester Gum H (trade name)", and indomethacin was added in place of diclofenac sodium.

### Example 8

A skin patch (plaster) was produced by preparing a base by a similar procedure to Example 1 except that the amount of liquid paraffin added was 4162 g, the amount of N-methyl-2-pyrrolidone added was 180 g, the amount of 1-menthol added was 135 g, and the amount of dibutylhydroxytoluene added was 22.5 g.

### Example 9

A skin patch (plaster) was produced by preparing a base by a similar procedure to Example 1 except that the amount of N-methyl-2-pyrrolidone added was 180 g, and that 90 g of diclofenac sodium was further added.

### Example 10

A skin patch (plaster) was produced by preparing a base by a similar procedure to Example 9 except that ketoprofen was added in place of diclofenac sodium, and "KE-100 (trade name)" was added in place of "Ester Gum H (trade name)".

### Example 11

A skin patch (plaster) was produced by preparing a base by a similar procedure to Example 9 except that the amount of liquid paraffin added was 3654 g, the amount of N-methyl-2-pyrrolidone added was 360 g, felbinac was added in place of diclofenac sodium, and 36 g of citric acid was further added.

### Example 12

A skin patch (plaster) was produced by preparing a base by a similar procedure to Example 11 except that the amount of 1-menthol added was 135 g, the amount of dibutylhydroxytoluene added was 45 g, 180 g of a hydrogenated oil was added, "KE-100 (trade name)" was added in place of "Ester Gum H (trade name)", and diclofenac sodium was added in place of felbinac.

### Example 13

A skin patch (plaster) was produced by preparing a base by a similar procedure to Example 1 except that the amount of "D-KX401CS (trade name)" added was 3150 g, the amount of "Ester Gum H (trade name)" added was 2700 g, the amount of liquid paraffin added was 3150 g, and further, N-methyl-2-pyrrolidone, 1-menthol, and dibutylhydroxytoluene were not added.

### Example 14

A skin patch (plaster) was produced by preparing a base by a similar procedure to Example 1 except that the amount of "D-KX401CS (trade name)" added was 1800 g, the amount of liquid paraffin added was 3510 g, the amount of N-methyl-2-pyrrolidone added was 180 g, the amount of 1-menthol added was 90 g, 180 g of hydrogenated oil and 90 g of felbinac were added, dibutylhydroxytoluene was not added, and 3150 g of "KE-100 (trade name)" was added in place of "Ester Gum H (trade name)".

### Example 15

A skin patch (plaster) was produced by preparing a base by a similar procedure to Example 1 except that the amount of "D-KX401CS (trade name)" added was 2700 g, the amount of "Ester Gum H (trade name)" added was 1350 g, the amount of liquid paraffin added was 4500 g, the amount of N-methyl-2-pyrrolidone added was 360 g, further, 90 g of loxoprofen sodium was added, and 1-menthol and dibutylhydroxytoluene were not added.

### Example 16

A skin patch (plaster) was produced by preparing a base by a similar procedure to Example 1 except that the amount of "D-KX401CS (trade name)" added was 1350 g, the amount of "Ester Gum H (trade name)" added was 2700 g, the amount of liquid paraffin added was 3960 g, 900 g of "D-1107 (trade name)" and 90 g of ketoprofen were further added, and N-methyl-2-pyrrolidone, 1-menthol, and dibutylhydroxytoluene were not added.

A summary of the compositions of Examples 1 to 16 are as shown in Table 4.

**Table 4**

| | SIS | Tackifier | LP | Hydrogenated oil | Optional Ingredient | | | | |
|---|---|---|---|---|---|---|---|---|---|
| | | | | | Solubilizer and Transdermal Absorption Promoting Agent of Drug | | | | Medicinal Agent |
| | | | | | NMP | L-MEN | BHT | Others | |
| Example 1 | 22/78 2250g | EGH 2250g | 3870g | - | 270g | 270g | 90g | - | - |
| Example 2 | 22/78 2250g | EGH 2250g | 3780g | 180g | 270g | 90g | 90g | - | DFNa 90g |
| Example 3 | 22/78 2250g | EGH 2250g | 3285g | 180g | 360g | 90g | 90g | Isoste aric Acid 360g Citric Acid 45g | DFNa 90g |
| Example 4 | 15/85 2250g | KE 2250g | 3870g | - | 270g | 270g | 90g | - | - |
| Example 5 | 15/85 2250g | EGH 2250g | 3780g | - | 270g | 270g | 90g | - | DFNa 90g |
| Example 6 | 22/78 2250g | ECH 2250g | 3780g | - | 270g | 270g | 90g | - | KP 90g |
| Example 7 | 22/78 2250g | ARKON 2250g | 3780g | - | 270g | 270g | 90g | - | ID 90g |
| Example 8 | 22/78 2250g | EGH 2250g | 4162g | - | 180g | 135g | 22.5g | - | - |
| Example 9 | 22/78 2250g | EGH 2250g | 3870g | - | 180g | 270g | 90g | - | DFNa 90g |
| Example 10 | 22/78 2250g | KE 2250g | 3870g | - | 180g | 270g | 90g | - | KP 90g |
| Example 11 | 22/78 2250g | EGH 2250g | 3654g | - | 180g | 270g | 90g | Citric Acid 36g | FB 90g |
| Example 12 | 22/78 2250g | KE 2250g | 3654g | 180g | 360g | 135g | 45g | Citric Acid 36g | DFNa 90g |
| Example 13 | 22/78 3150g | EGH 2700g | 3150g | - | - | - | - | - | - |
| Example 14 | 22/78 1800g | KE 3150g | 3510g | 180g | 180g | 90g | - | - | FB 90g |
| Example 15 | 22/78 2700g | EGH 1350g | 4500g | - | 360g | - | - | - | RPNa 90g |
| Example 16 | 22/78 1350g 15/85 900g | EGH 2700g | 3960g | - | - | - | - | - | KP 90g |

| | | | | | | | | | |
|---|---|---|---|---|---|---|---|---|---|
| SIS: SIS block copolymer EGH: Ester Gum H (rosin ester derivative) KE: KE-100 (rosin ester derivative) ARKON: ARKON P-100 (alicyclic saturated hydrocarbon resin) BHT: dibutylhydroxytoluene LP: liquid paraffin NMP: N-methyl-2-pyrrolidone L-MEN: 1-menthol KP: ketoprofen DFNa: diclofenac sodium ID: indomethacin FB: felbinac LXNa: loxoprofen sodium | | | | | | | | | |

Examples 1 to 16, and commercially available skin patches containing an agglutinant of a SIS block copolymer were evaluated based on self-adhesion property (handleability), skin stimulatory property, adhesion to skin, and adhesion to skin in the presence of moisture (water resistance) by measuring physical characteristic values of the base (tanδ (angle of lag) and glass transition temperature (Tg)).

### Test Example 1: Measurement of Tanδ and Glass Transition Temperature (Tg)

Using a rheometric dynamic analyzer "Dynamic Analyzer RDAIII (trade name)" (manufactured by Rheometric Scientific, Inc.), the dynamic shear modulus (G') and the like of the agglutinant was measured.

Specifically, the base was first scraped from the base layer of the skin patch, and then about 65 mg of the base was sandwiched in a cylindrical jig. The stress generated when a strain of 1% at a frequency of 6.2832 (rad/s) was applied while raising the temperature of the base from -70°C to 120°C at a rate of 5°C/min was measured and plotted every 12 seconds. Parallel plates having a diameter of 8.0 mm were used and the clearance (Gap) was set to be 1.298 mm. G' and G" were calculated using PC software (Orchestrator Ver. 6.5.6; produced by Rheometric Scientific, Inc.). Tanδ (angle of lag) (= G"/G') was similarly calculated using PC software (Orchestrator Ver. 6.5.6; produced by Rheometric Scientific, Inc.). Additionally, the glass transition temperature (Tg) value was determined from a profile curve of tanδ.

### Measurement Condition

- Frequency: 6.2832 (rad/s).
- Temperature: -70°C to 120°C (initially set at -70°C, followed by increasing the temperature to 120°C at a rate of 5°C/min).
- Plot of measurement: measured and plotted every 12 seconds.
- Plate: parallel plates with a diameter of 8.0 mm.
- Clearance (Gap): 1.298 mm.
- Amount of strain: 1%.

Measurements were made on commercial skin patch products a to g, and on the bases obtained in Examples 1 to 16. The tanδ (angle of lag) value at glass transition temperatures of 45°C and 32°C was as shown in Table 5.

**Table 5**

| | Glass Transition Temperature (°C) | tanδ @ 45 °C | tanδ @ 32 °C |
|---|---|---|---|
| Commercial Product a | -46.84 | 0.26 | 0.27382 |
| Commercial Product b | -52.82 | 0.31 | 0.30100 |
| Commercial Product c | -46.85 | 0.21 | 0.23650 |
| Commercial Product d | -46.86 | 0.41 | 0.31273 |
| Commercial Product e | -40.89 | 0.40 | 0.51035 |
| Commercial Product f | -22.90 | 0.11 | 0.17114 |
| Commercial Product g | -46.85 | 0.56 | 0.40638 |
| Example 1 | -40.87 | 0.20 | 0.26100 |
| Example 2 | -40.87 | 0.21 | 0.26254 |
| Example 3 | -40.88 | 0.24 | 0.28606 |
| Example 4 | -43.00 | 0.18 | 0.28100 |
| Example 5 | -43.00 | 0.20 | 0.26094 |
| Example 6 | -40.87 | 0.17 | 0.26100 |
| Example 7 | -40.87 | 0.19 | 0.26100 |
| Example 8 | -37.9 | 0.16 | - |
| Example 9 | -39.8 | 0.17 | - |
| Example 10 | -38.8 | 0.18 | - |
| Example 11 | -40.8 | 0.19 | - |
| Example 12 | -37.9 | 0.23 | - |
| Example 13 | -36.0 | 0.24 | - |
| Example 14 | -42.0 | 0.22 | - |
| Example 15 | -37.9 | 0.23 | - |
| Example 16 | -42.5 | 0.24 | - |

### Test Example 2: Evaluation of Handleability

After peeling the liner of the skin patch, the base face of each of the aforementioned commercial products a to g, and the skin patches (10 cm x 7 cm) of Examples 1 to 16 were folded into two so that the two halves made contact with each other. Sensory evaluation was determined on the ease of release of peeling according to the following standards.

### Evaluation Standards

A: separable without resistance.
B: separable accompanied by some resistance.
C: separable with some degree of difficult.
D: separable with difficulty in returning to the original state, accompanied by partial detachment of the base layer.

### Test Example 3: Evaluation of Skin Stimulatory Property (Pain in Peeling)

The commercial products a to g, and the skin patches (10 cm x 7 cm) of Examples 1 to 16 were applied to an area on the right upper arm of five monitors. The pain felt upon peeling each skin patch from the area on the right upper arm was evaluated according to the following standards.

### Evaluation Standards

A: no pain felt at all.
B: some pain felt.
C: pain felt.
D: strong pain felt.

### Test Example 4: Evaluation of Adhesion to Skin

The commercial products a to g, and the skin patches (10 cm x 7 cm) of Examples 1 to 16 were applied to an area on the right upper arm of five monitors. The state of application (state of detachment) on the skin of each skin patch after 8 hours was visually observed. The skin patches with their entire face attached to the skin were deemed as "no release", and evaluation was made according to the following standards.

### Evaluation Standards

A: no release.
B: very slight release.
C: somewhat broad release on all four corners and the like.
D: release found over a wide area, or considerable shift in the application site.

### Test Example 5: Evaluation of Adhesion to Skin in the Presence of Moisture (Water Resistance)

The commercial products a to g, and the skin patches (10 cm x 7 cm) of Examples 1 to 16 were applied to an area on the right upper arm of five monitors after spraying purified water on the respective area. Followed by employing the test procedure according to Test Example 4 to evaluate the adhesion to skin of each skin patch.
Results of evaluation by Test Examples 2 to 5 are shown in Table 6.

**Table 6**

| | Test Example 2 Handleability | Test Example 3 Pain in Peeling | Test Example 4 Adhesion to Skin | Test Example 5 Adhesion to Skin in the Presence of Moisture |
|---|---|---|---|---|
| Commercial Product a | C | D | A | B |
| Commercial Product b | C | D | A | - |
| Commercial Product c | B | C | A | - |
| Commercial Product d | D | - | B | - |
| Commercial Product e | B | C | A | - |
| Commercial Product f | C | - | B | - |
| Commercial Product g | D | C | C | D |
| Example 1 | A | A | A | A |
| Example 2 | A | A | A | A |
| Example 3 | A | A | A | A |
| Example 4 | B | A | A | A |
| Examples 5 | B | A | A | A |
| Example 6 | A | A | A | A |
| Example 7 | A | A | A | A |
| Example 8 | A | A | A | A |
| Example 9 | A | A | A | A |
| Example 10 | A | A | A | A |
| Example 11 | A | A | A | A |
| Example 12 | A | A | A | A |
| Example 13 | A | A | B | B |
| Example 14 | A | A | A | A |
| Example 15 | A | A | A | A |
| Example 16 | A | A | B | B |

The bases produced according to Examples 1 to 16 exhibited substantially similar temperature dependency of tanδ (angle of lag) to the illustration of temperature dependency of tanδ (angle of lag) shown in Fig. 1 or Fig. 2. Specifically, as shown in Table 5, the base had a glass transition temperature of no lower than -45°C and no greater than -35°C, and had a tanδ (angle of lag) at 45°C of no greater than 0.25. In addition, the tanδ (angle of lag) at 32°C was no less than 0.20 and no greater than 0.45. In contrast, the commercial products a to g did not meet the requirements of including a base having a glass transition temperature of no less than - 45°C and no greater than -35°C, and having a tanδ (angle of lag) at 45°C of no greater than 0.25.

On the other hand, as shown in Table 6, it was demonstrated that the skin patches produced in Examples 1 to 16 could improve the handleability, adhesion to skin, and water resistance, furthermore the amount of stimulation provided to the skin could be reduced. In contrast, none of the commercial products a to g could improve the handleability, adhesion to skin, and water resistance, furthermore the amount of stimulation provided to the skin could not be reduced.

From the foregoing, it was revealed that the water resistance and handleability can be improve, and the stimulation given to the user could be reduced by allowing the base that includes an SIS block copolymer to have a glass transition temperature (Tg) of no lower than -45°C and no higher than -35°C, and have a tanδ (angle of lag) value determined by measurement of dynamic viscoelasticity (frequency: 6.2832 (rad/s)) at 45°C being no greater than 0.25.

## Claims

1. A skin patch comprising a flexible backing, and a base layer laminated on the backing, wherein the base layer comprises a styrene-isoprene-styrene block copolymer, having a glass transition temperature (Tg) of no less than -45°C and no greater than -35°C, and having a tanδ (angle of lag) value determined by measurement of dynamic viscoelasticity (frequency: 6.2832 (rad/s)) at 45°C being no greater than 0.25.

2. The skin patch according to claim 1, wherein the tanδ (angle of lag) value is no less than 0.12 and no greater than 0.25.

3. The skin patch according to claim 2, wherein the tanδ (angle of lag) value is no less than 0.15 and no greater than 0.25.

4. The skin patch according to any one of claims 1 to 3, wherein the base layer further comprises a tackifier, and a plasticizer.

5. The skin patch according to claim 4, wherein the content of the styrene-isoprene-styrene block copolymer is no less than 10% by weight and no greater than 40% by weight; the content of the tackifier is from 10 to 35% by weight; and the content of the plasticizer is no less than 20% by weight and no greater than 60% by weight based on the weight of the entire base layer.

6. The skin patch according to any one of claims 1 to 5, wherein the styrene-isoprene-styrene block copolymer has a weight ratio of styrene to isoprene (styrene/isoprene) no less than 20/80 and no greater than 25/75.
